# EUROPÄISCHE PATENTSCHRIFT

(11) **EP 0 495 132 B1**
(45) Veröffentlichungstag und Bekanntmachung des Hinweises auf die Patenterteilung: **18.06.2003**
(21) Anmeldenummer: 91914287.7
(22) Anmeldetag: 02.08.1991
(51) Int. Cl.: A61H 39/00, A61M 37/00, A61K 9/00, A61N 1/16

(54) **VORRICHTUNG ZUM ENERGIEINFORMATIONSAUSTAUSCH ZWISCHEN OBJEKTEN**
DEVICE FOR ENERGY-INFORMATION EXCHANGE BETWEEN OBJECTS
DISPOSITIF D'ECHANGE D'INFORMATIONS D'ENERGIE ENTRE DES OBJETS

(30) Priorität: 02.08.1990 SU 4876365; 15.08.1990 SU 4875839; 15.11.1990 SU 4883074
(43) Veröffentlichungstag der Anmeldung: 22.07.1992
(73) Patentinhaber: LUPICHEV, Nikolay Lvovich, Moscow, 117419 (SU)
(72) Erfinder: LUPICHEV, Nikolay Lvovich, Moscow, 117419 (SU)
(74) Vertreter: Patentanwälte Zellentin & Partner
(86) Internationale Anmeldenummer: SU9100161
(87) Internationale Veröffentlichungsnummer: WO92002201

(56) Entgegenhaltungen:
- WO-A-91/10450
- CH-A- 670 766
- DE-A- 3 413 540
- DE-A- 3 521 779
- DE-A- 3 731 560
- DE-B- 2 659 115
- FR-A- 2 236 514
- AM. J. ACUPUNCTURE Bd. 8, Nr. 2, April 1980, Seiten 97 - 104 VOLL 'The Phenomenon of Medicine Testing in Electroacupuncture According to Voll'

## Beschreibung

Die Erfindung betrifft ein Gerät zur Übertragung einer Medikamentenwirkung bei Elektroakupunkturverfahren zwischen einer informativen Substanz und einem Träger mit einem eine Stromquelle aufweisenden, elektrischen Schaltkreis, der zwei Kontaktelemente umfaßt, von denen eines zum Zusammenwirken mit der Substanz und das andere mit dem Träger geeignet ist.

Es ist eine Einrichtung zur Übertragung der Medikamentenwirkung , naemlich ein Gerät zur Übertragung von Heileigenschaften der Medikamente von R.Voll, welches beim medikamentösen Testen angewandt wird, bekannt. Es weist eine passive Elektrode und eine aktive Elektrode - einen Taster - auf, die durch eine elektrische Kette miteinander verbunden sind, in welche ein Ohmmeter und eine Gleichstromquelle eingeschaltet sind. Das Gerät enthält ebenfalls einen Platz zur Unterbringung von Ampullen mit Medikamenten, welcher an die elektrische Kette angeschlossen ist.

Das Gerät wird folgenderweise benutzt. Man stellt eine informative substanz - eine Ampulle mit einem zur Heilung geeigneten Arzneimittel - auf diesen Platz; die passive Elektrode wird vom Patienten in seiner Hand gedrückt (als Empfänger der Information dient hier das Akupunktursystem des Patienten) und mit der aktiven Elektrode - dem Taster - drückt der Arzt auf den zu untersuchenden Akupunkturpunkt des Patienten. Wenn, die Madel des Ohmmeters auf die Kontrollmake kommt, wird das so verstanden, dass das Arzneimittel zur Heilung der Erkrankung des Patienten gesignat ist. Wenn es eine Abweichung von dieser Kontrollmarke gibt, wird die Ampulle mit dem Arzneimittel auf dem Platz durch eine andere Ampulle ersetzt. Man ersetzt die Ampulle mit den Arzneimitteln, bis die Nadel des Ohmmeters auf die Kontrollmarke kommt. Man verabreicht dieses Arzneimittel dem Patienten durch spritzen oder Einnehmen.

Man muss aber sagen, dass ein Satz von Medikamenten, welcher zum Testen unter Einsetzung dieses bekannten Gerätes benutzt wird, sehr teuer ist, und da das Arzneimittel während des Heilungsprozesses in der Regel mehrmals eingenommen werden, ist die Heilung ebenfalls sehr teuer.

Es ist ausserdem eine zolche Einrichtung bekannt, bei der ein objekt als Informationsquelle und ein zweites Objekt als Informationsempfänger dient; die Einrichtung enthält einen elektrischen Kreis, welcher eine Speisequelle, und das erste und das zweite Kontaktelement einschliesst, die an den elektrischen Kreis angeschlossen sind (das erste Kontaktelement wirkt mit der Informationsquelle zusammen und das zweite Kontaktelement - mit dem Informationsempfänger) (s. die DE-A 3413540). In dieser Einrichtung stellen die Kontaktelemente Platten aus einem stromleitenden Material dar.

Die Einrichtung gestattet es, die Information von der Ampulle mit einem teueren Arzneimittel auf einen beliebigen billigen Träger, beispielsweise auf eine physiologische Lösung oder auf ein Magnetband zu übertragen, der nachträglich dem Kranken entweder eingeführt oder auf den Akupunkturpunkt des Kranken aufgeklebt wird (das Magnetband).

Man benutzt diese Einrichtung folgendermaßen: Auf eine Eingangskontaktplatte wird eine Ampulle mit einem Arzneimittel gestellt, während auf die andere Platte, die zum Zusammenwirken mit dem Informationsempfänger geeignet ist, ein Träger gebracht wird. Man schliesst den Stromkreis mittels eines Schalters und stellt mit Hilfe eines Potentiometers die Größe der in den Kreis zu gebenden Gleichspannung von der Quelle ein, welche das Durchfließen des elektrischen Stromes im Kreis über die Eingangs- und Ausgangskontaktplatten gewährleistet. Dabei wird durch die Verwendung der Energie der Speisequelle die Übertragung der Eigen schaften des Arzneimittels auf den Träger realisiert.

Die Energie der Speisequelle ist in der bekannten Einrichtung nicht effektiv genug genutzt; es sind bedeutende Energieverluste, besonders auf der Ausgangsplatte, zu verzeichnen. Die Ausstrahlung von der Ausgangsplatte kommt in der Regel nur teilweise auf den Träger. Da die Bewegung elektrischer Ladungen in der Platte in jedem ihrer Teile erfolgen kann, ist die Strahlungsleistung der Platte, welche auf den Träger übertragen wird, nicht hoch genug.

Im Zusammenhang damit erreichen den Träger alle erforderlichen Eigenschaften der Informationsquelle überhaupt nicht oder er bekommt diese in einem bedeutend geringerem Masse, was die Möglichkeiten der Therapie sowie die Möglichkeit, unterschiedliche Potenzen von Homöopathie-Präparaten zu erzielen, wesentlich einschränkt.

Der Erfindung ist eine Aufgabe zugrundegelegt, ein Gerät zur übertragung für Medikamentenrichtung bei Electroakupunkturverfahren zu schaffen, in welcher mindestens ein Kontaktelement, beispielsweise ein zum Zusammenwirken mit einem Informations-empfänger geeignetes Kontaktelement solch eine Konstruktion hat, die die Formierung eines konzentrierten gerichteten Informationsstromes gewährleistet, so daß es ermöglicht ist, diese Information in möglichst vollem Umfang auf den Träger zu übertragen, eine effektivere Nutzung der Energie der Speisequelle zu erzielen sowie zu gestatten, Arzneimittel auf beliebigen Trägern, in manchen Fällen auch mit einer stärkeren Wirkung als die Ausgangsträger, welche als informative substanzen verwendet werden, zu gewinnen.

Diese Aufgabe wird dadurch gelöst, daß das zum Zusammenwirken mit dem Träger geeignete Kontaktelement als Induktionsspule mit mindestens einer Wicklung ausgebildet ist, die in den elektrischen Schaltkreis eingeschaltet ist.

Es ist zweckmäßig, um den Wirkungsgrad zu erhoehen, die Induktionsspule mit mindestens einer zusätzlichen Wicklung auszurüsten, deren Windungen zur Grundwicklung koaxial angeordnet sind.

Um die Qualität der Strahlung der Information zu erhöhen, ist die Spule in Form eines Torus ausgebildet.

Man kann auch zu diesem Zweck die Spule in Form einer flachen Spirale ausbilden.

Zu einer vollständigen Abnahme der Information ist es möglich, das zum Zusammenwirken mit der informativen substanz geeignete Kontaktelement in Form eines Trichters auszubilden, an dessen Spitze eine Stromabnahmestelle aus einem stromleitenden Material vorgesehen ist.

Es ist zweckmäßig, entlang der Drehachse der Induktions-spule einen Kern vorzusehen.

Es wäre dabei wünschenswert, vom der Kern die Form eines Stabes aufweist.

Um die Möglichkeit zu schaffen, den Informationsempfänger in einem beträchtlichen Abstand von der Einrichtung anzuordnen, wäre es wünschenswert, ein Ende des Stabes spitz auszubilden.

Um die Möglichkeit zu schaffen, den Zustand des Akupunktursystems des Menschen zu korrigieren, wäre es zweckmäßig, den Stab mit dem elektrischen Kreis elektrisch zu verbinden.

Um die Einwirkung auf das Akupunktursystem regeln zu können, ist es wünschenswert, den Stab mit der Möglichkeit einer Verschiebung entlang seiner Achse anzuordnen.

Zu demselben Zweck kann man das Material für den Stab unter Berücksichtigung seiner Identität mit dem Material eines der Homöoapatie-Präparate, welche zur Heilung dieser Krankheit vorgeschrieben sind, wählen.

Zur Verstärkung des Energie-Informationssignals ist es möglich, einige Einrichtungen zum Energie-Informationsaustausch zwischen den Objekten, von denen jede einen elektrischen Kreis mit einer Energiequelle und zwei daran angeschloßenen Kontaktelementen, und von welchen eines die Induktionsspule darstellt, in einer Reihe nacheinander anzuordnen, so daß das Kontaktelement jeder nachfolgenden Einrichtung, die zum Zusammenwirken mit der informativen Substanz geeignet ist, in der Nähe der Stirn der Induktionsspule der vorangehenden Einrichtung angeordnet wäre und die Drehachse dieser Spule durch dieses Kontaktelement verlaufen könnte.

Dank der Ausbildung des Kontaktelementes, welches zum Zusammenwirken mit der informativen Substanz geeignet ist, in Form einer Induktionsspule wird die Formierung eines gerichteten konzentrierten Informationsstroms entlang der Drehachse der Spule erreicht. Indem man die Informationsempfänger - Träger - auf dem Wege der Verbreitung dieses Stromes anordnet, gelingt es, die Träger mit einer hohen Heilwirkung und manchmal mit einer Wirkung, die die Heilwirkung des ausgänglichen Arzneimittels übertrifft, zu erzielen. Durch die Verminderung der Verluste bei der Übertragung der Information vom strahlenden Kontaktelement auf den Empfänger wird einer höhter Wirkungsgrad gegenüber bekannten Einrichtungen mit entsprechender Zweckbestimmung erzielt.

Es ergibt sich eine hohe Qualität der Übertragung der Eigenschaften der Arzneimittel auf die Träger, die Zeit zur Durchführung des Energie-Inforamtionsaus-tausches zwischen der Quelle und dem Träger wird verkürzt, und es gibt viele Möglichkeiten zum Regeln der Parameter des Energie-Informationsaustausches. Eine der Varianten gestattet es, eine Korrektur des Zustandes des Akupunktursystems des Menschen vorzunehmen.

Weiterhin wird die Erfindung durch die Beschreibung konkreter Ausführungsbeispiele und anhand von Zeichnungen näher erläutert. Es zeigen.
Fig.1 eine prinzipielle elektrische Schaltung des Geräts zur übertragung für Medikamentenwirzung bei Elektroakupunkturverfahren;
Fig.2 das gerät von Fig.1 mit bonusförmiges Induktionsspule gemäß des Erfindung;
Fig.3 eine Ausführungsvariante mit einer Induktionsspule in Form einer flachen Spirale;
Fig. 4 eine Ausführungsvariante mit einer Induktionsspule in Form einer Raumspirale;
Fig.5 das Gerät von Fig.2 mit einem stabförmigen Kern und dem ersten Kontaktelement in Form eines Trichters;
Fig.6 einen Abschnitt A von Fig.5 vergrößert entsprechend eines Ansicht gemäß dem Pfeil B;
Fig.7 eine prinzipislle elektrische Schaltung des Geräts das zur Kontrolle des Zustandes des Akupunktursystems des Menschen geeignet ist;
Fig.8 dieselbe elektrische Schaltung wie von Fig. 7 für eine Variante mit einem Elektroimpulsgenerator mit Wechselfrequenz; und
Fig.9 eine prinzipielle elektrische Schaltung eines Verstärkers für das Energie-Informations-signal.

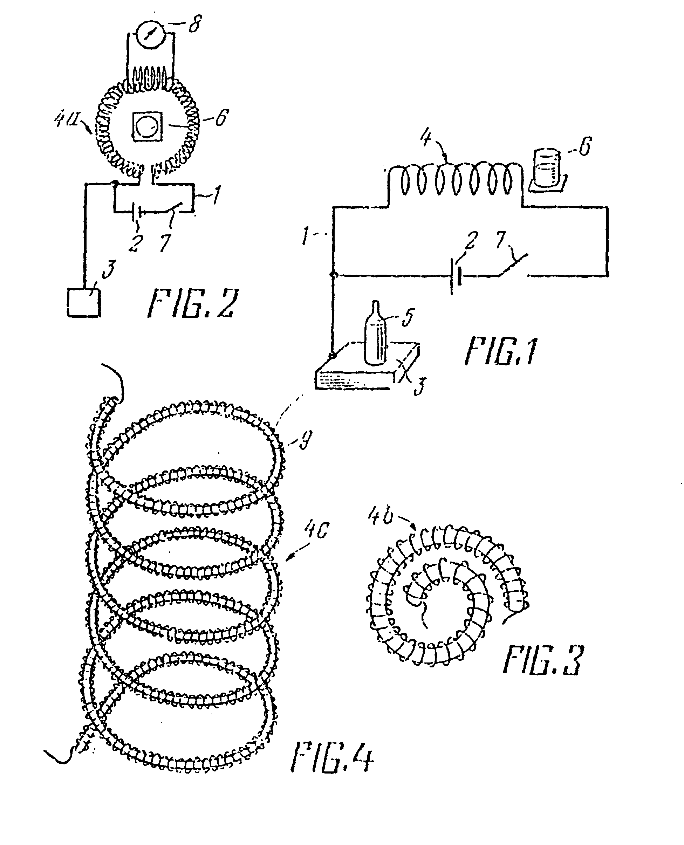

Das auf Fig.1 gezeigte Gerät enthält einen elektrischen Kreis 1, welcher eine Speisequelle 2, in diesem Fall eine Gleichstromquelle, einschließt. Das Gerät enthält ausserdem zwei Kontaktelemente 3 und 4, von welchen das Kontaktelement 3 in Form einer Platte aus einem elektrisch leitenden Material , beispielsweise Al, ausgebildet ist, die an den Kreis 1 angeschlossen ist, während das Kontaktelement 4 eine Induktionsspule darstellt, die in den Kreis 1 in Reihe geschaltet ist. Das Kontaktelement 3 ist zum Zusammenwirken mit einer informativen Substanz, als welche zum Beispiel eine Ampulle 5 mit einem Arzneimittel dient, geeignet. Das Kontaktelement 4, weiterhin die Induktionsspule 4 genannt, eignet sich zum Zusammenwirken mit einem Informationsempfänger 6, beispielsweise mit einem Träger (Wasser, Zucker), oder mit dem Akupunktursystem des Menschen.

Der elektrische Kreis 1 ist ausserdem mit einem Schalter 7 versehen.

In Fig.2 ist eine Variante desselben Geräts wie in Fig.1 dargestellt; aber bei dieser beanspruchten Ausführung weist die Induktionsspule 4a die Form eines Torus auf und das Gerät ist mit einem Stromm messer, der die Spule 4a durchfliesst, nämlich mit einem Ampèremeter 8, welches mit der Induktionsspule 4a induktiv verbunden ist, versehen.

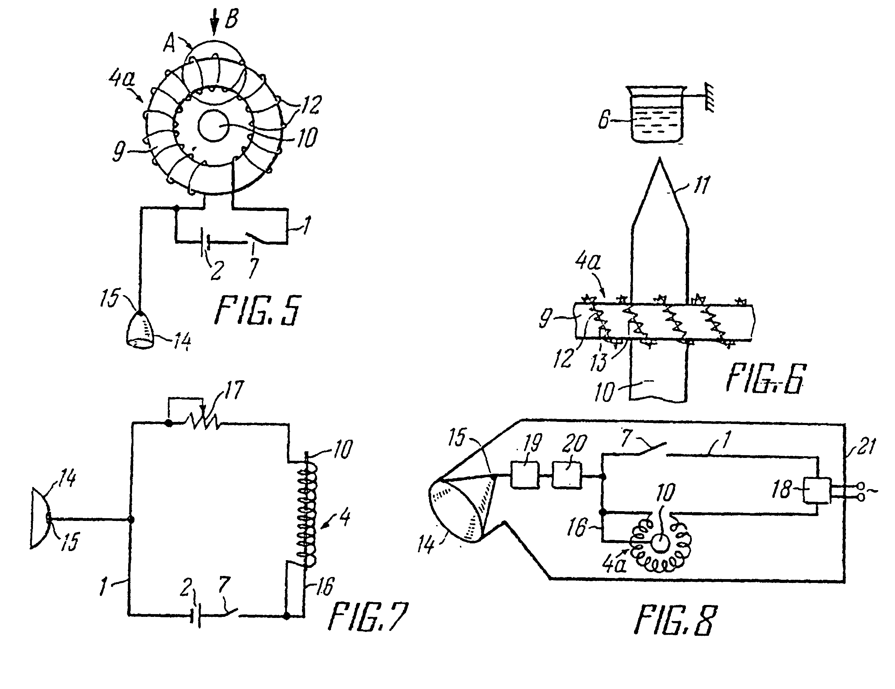

Fig.3 zeigt eine Ausführungsvariante der Induktionsspule 4b, die die Form einer flachen Spirale aufweist und mit einem Ferromagnetkern 9 versehen ist, der ebenfalls die Form einer flachen Spirale aufweist.

Die in Fig.4 gezeigte Induktionsspule 4c ist in Form einer Raumspirale mit einer ersten Umdrehung ausgebildet und ihr ferromagnetischer Kern 9 weist ebenfalls die Form einer derartigen Raumspirale auf.

Die Induktionsspule kann auch in Form einer Raumspirale mit zwei, drei usw. Umdrehungen ausgebildet sein, wozu man die in Fig.4 gezeigte Spule 4c in eine Raumspirale noch einmal umwickelt und dadurch eine Raumspirale mit zwei Umdrehungen bekommt; dann wickelt man die letztere Spirale noch einmal usw.

Die spiralartige Induktionsspule kann sowohl als eine einen Ferromagnetkern aufweisende Spule als auch ohne Kern ausgebildet sein.

Außerdem kann erfindungsgemäß jede beliebige in Fig.1 bis 4 gezeigte Ausführungsvariante der Induktivionsspule zusätzliche Wicklungen aufweisen, wobei die Windungen jeder darauffolgenden Wicklung zum Leiter der vorangehenden Wicklung koaxial angeordnet sind, so daß dieser Leiter für die darauffolgende Wicklung die Rolle eines Kerns spielt.

Die Fig.5, 6 zeigen eine Ausführungsvariante , in welcher im Gegensatz zu der in Fig.2 gezeigten Variante um die Drehachse der Spule 4a in der Drehachse des Torus) ein zusätzlicher Kern vorgesehen ist, der die Form eines Stabes 10 aufweist. Das Arbeitsende 11 des Stabes 10 tritt aus den Grenzen der Stirn der Spule 4a heraus und ist spitz ausgebildet. Aus der Fig.6, wo der Abschnitt A der Spule 4a vergrößert dargestellt ist, ist ersichtlich, daß die Spule 4a außer der Grundwicklung 12 eine zusätzliche Wicklung 13 aufweist, deren Windungen zur Grundwicklung 12 koaxial angeordnet sind.

Ebenfalls muß gesagt werden, daß in dieser Ausführungsvariante im Gegensatz zu der oben beschriebenen Ausführungsvariante das Kontaktelement, das zum Zusammenwirken mit einer Informativen Substanz geeignet ist, die Form eines Trichters 14 aufweist, an dessen Spitze eine Stromabnahmestelle 15 vorgesehen ist, die aus einem stromleitenden Material, beispielsweise aus Al, gebildet ist. Die Stelle 15 ist an den elektrischen Kreis 1 elektrisch angeschloßen.

Fig.7 zeigt eine Ausführungsvariante, in welcher der Kern, der um die Drehachse der Spule 4 angeordnet ist, durch einen Leiter 16 an den Kreis 1 elektrisch angeschlossen ist. Der Stab 10 hat in diesem Falle kein spitzes Ende, außerdem kann er entlang seiner Achse, zum Beispiel mittels einer Meßschraube (auf der Zeichnung nicht gezeigt) verschiebbar angeordnet sein, was es gestattet, die Anzahl der Windungen der Spule 4, die mit dem Stab 10 in Wechselwirkung stehen, zu ändern.

Bei dieser Ausführungsvariante ist in den Kreis 1 auch ein regelbarer ohmscher Widerstand eingeschaltet.

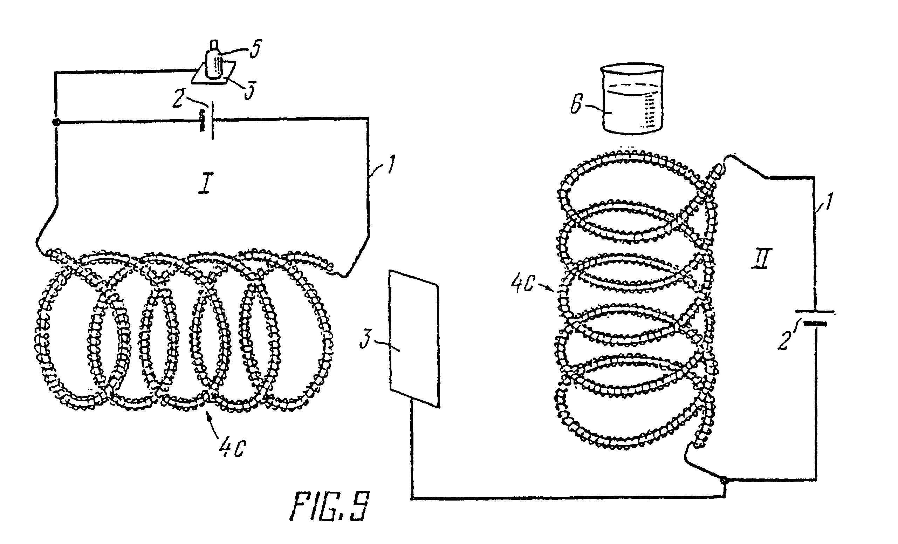

Bei der auf Fig.8 dargestellten Variante wird im Gegensatz zu der in Fig.7 gezeigten Variante im elektrischen Kreis 1 als Speisequelle ein Generator 18 für elektrischewechselströme impulse benutzt und die Induktionsspule 4a weist die Form eines Torus auf. Ferner sind in den elektrischen Kreis zwischen des Stromabnahmestelle 15 des Trichters 14 und dem Kreis 1 ein in Reihe geschaltetes Filter 19 und ein Verstärker 20 eingeschaltet.

Als Filter 19 kann ein beliebiges bekanntes elektrisches Filter, zum Beispiel RC oder RLC mit regelbaren Parametern, um die übertragungsfrequenz ändern zu können, angewendet werden.

Fig.8 zeigt die Einrichtung, die in einem Gehäuse 21 angeordnet ist.

Fig.9 zeigt eine prinzipielle elektrische Schaltung des Verstärkers des Geräts, das einige, im zu beschreibenden Beispiel zwei Stufen I, II enthält, von welchen jede einen elektrischen Kreis 1 mit einer Speisequelle 2 und zwei Kontaktelemente 3 und 4 einschließt. Das Kontaktelement 3 stellt eine Platte aus einem elektrisch leitenden Material dar, als Kontaktelement 4 können beliebige oben beschriebene Induktionsspulen, zum Beispiel eine Spiralspule 4c dienen. Die Stufen I, II usw. sind in Reihe nacheinander angeordnet, so daß das Kontaktelement 3 der Stufe II (der darauffolgenden Stufe) in der Nähe der Stirnseite der Spule 4c der Stufe I (der vorangehenden Stufe) angeordnet ist und sich mit der Drehachse der Spule 4c kreuzt.

Dabei wäre es wünschenswert, daß die Plattenebene (des Kontaktelementes 3) der Drehachse der Spule 4c senkrecht angeordnet wäre.

Der Verstärker ist zusammen mit dem erfindungsgemäßen Gerät gezeigt, dessen Schaltung in Fig.1 dargestellt ist. Es könnte aber zum Aufbau eines solchen Verstärkers ein beliebiges Schema des Geräts das in Fig. 2, 5, 7 oder 8 gezeigt ist, angewendet werden.

Das Kontaktelement, das zum Zusammenwirken mit der informativen substanz geeignet ist, ist in Form eines Trichters ausgebildet. In diesem Fall muß man dieses mit seinem sich verjüngendes Ende nach vorne im Laufe des Informationsstromes der Stirn der Spule 4c der Stufe I gegenüberliegend anordnen, so daß die Drehachse der Spule 4c über die Stromabnahmestelle des Trichters verläuft.

Dieses Kontaktelement kann auch die Form eines Stabes, der aus einem stromleitenden Material ausgebildet ist, haben; man erindet den Stab um die Achse der Spule 4c der Stufe I in ihrem Inneren und schaltet diesen an den Kreis 1 der Stufe II elektrisch .

Die Einrichtung zum Energie-Informationsaustausch arbeitet wie folgt.

Man setzt auf das Kontaktelement 3 (Fig.1) eine Ampulle 5 mit einem Arzneimittel, dessen Eigenschaften dem Träger 6 übertragen werden müssen. Als letzterer können z.B. Wasser, Zucker usw. dienen.

Als Träger kann auch das Akupunktursystem des Menschen dienen. Der Träger wird an einem Ständer gegenüber der Stirn der Induktionsspule in unmittelbarer Nähe dieser Stirn so befestigt, daß die Drehachse der Spule 4 über den Träger 6 verläuft. Man schließt den Schalter 7, wobei über den Kreis 1 der elektrische Strom zu fließen beginnt, der durch das Vorhandensein einer Speisequelle 2 im elektrischen Kreis 1 bedingt ist. Beim Fließsen des Stromes über die Wicklung der Spule 4 formiert die Induktivität entlang ihrer Drehachse einen konzentrierten Energie--Informationsstrom, der zum Träger 6 hin gerichtet ist und durch diesen fließt. Somit übt die Induktionsspule 4 die Funktion eines Strahles aus, welcher es gestattet, einen konzentrierten Informationsstrom zu formieren und dem Träger Heileigenschaften des Arzneimittels mit minimalen Verlusten zu übertragen.

Die Einsetzung einer toroidalen Induktionsspule 4a (Fig.2) gestattet es, den Wirkungsgrad der Einrichtung zu erhöhen. In derselben Spule wird der beim Fließsen des Stromes über ihre Wicklung entstehende Magnetstrom im Inneren des Torus geschlossen; im Raum, der vom Torus umfasst wird, ist die Intensität des Magnetfeldes jedoch gleich Null, der Magnetstrom fehlt, während der Informationsstrom gerade um die Drehachse der toroidalen Spule formiert wird. Wenn man den Träger über der Spule 4a so anordnet, daß die Drehachse der Spule durch den Träger verläuft, gelingt es, Störungen auszuschließen, die durch den Magnetstrom in den Energie-Informationsstrom eingetragen werden, und erlaubt es somit die Qualität der Übertragung der Eigenschaften der Arzneimittel auf die Träger sowie den Wirkungsgrad der Einrichtung zu erhöhen.

Die Benutzung einer flachen spiralförmigen Induktionsspule 4b (Fig.3) (ebenso wie einer spiralförmigen Raumspule 4c) (Fig.4) gestattet es, auf einer Fläche, die derjenigen Fläche, die besetzt ist, zum Beispiel von einem Torus, gleich ist, eine größere Zahl der Windungen anzubringen, was die magnetische Randspannung der Spule und dementsprechend die Menge der mit dieser Spule auszuscheidenden Energie zu erhöhen, ermöglicht. Dabei ist es vorteilhaft, die Spule 4c in Form eines Konus auszuführen, der sich in Richtung der Verbreitung des Energie-Informationsstromes verjüngt, was zu einer größeren Konzentration dieses Stromes beiträgt.

Die Formierung der Spiralen aus Ranmspiralen gestattet es, vom Informationsstrom aufeinanderfolgend ein Magnetfeld und dann einzelne Formanden des Energie-Informationsstromes zu trennen.

In der Ausführungsvariante, die in Fig.5, 6 gezeigt wird, bedingt das Vorhandensein eines in Form eines Stabes 10 ausgebildeten Kerns, der entlang der Drehachse der Spule 4a angeordnet ist, die Verstärkung der Intensität des Energie-Austausches zwischen den Objekten. Das spitze Ende 11 des Stabes 10 gestattet es, die Information auf eine bedeutend größere Weite auszustrahlen. So hat es die Benutzung eines spitzen Stabes 10 aus einem ferromagnetischen Material unter denselben gleichen Bedingungen gestattet, die Weite der Energie-Informationseinwirkung ungefähr um das 100-fache zu erhöhen. Dies ist besonders in den Fällen bequem, wenn als Informationsempfänger das Akupunktursystem des Menschen dient.

Das Material des Stabes 10 kann auch unterschiedlich sein. Bei der Herstellung eines Materials, welches nach seiner Zusammensetzung dem Material irgendeines Homöopathiemittels analog ist, das zur Heilung einer bestimmten Krankheit zugeordnet wird, gelingt es, die Intensität der Einwirkung auf den Patienten des als Informationsquelle zu verwendenden Arzneimittels zu erhöhen.

So wird das Homöopathie -Präparat Cuprum bei verschiedenen Neuralgie-Syndromen benutzt. Dementsprechend wird die Benutzung des Stabes 10 aus Kupfer bei der Heilung von Erkrankungen des Nervensystems zur Erhöhung der Intensität der Heilwirkung des Homöopathie -Präparats Cuprum beitragen.

Die Anwendung der zusätzlichen Windungen 13 gestattet es, die magnetische Randspannung der Induktionsspule 4a zu erhöhen, und trägt letzten Endes zur Erhöhung der Energieabgabe bei.

Die Verstärkung des Energie-Informationsaustausches zwischen den Objekten wurde auf experimentellem Wege mittels einer Elektropunktur-Messung der Akupunkturpunkte nach der P.Voll-Methode ausgewertet (R.Voll, The Phenomenon of medicine Testing in Electroacupuncture According to Voll, Amer. ε Acupuncture, v.8(2) p.97-104, 1980).

Die Intensität des Energie-Informationsaustausches läßt sich regeln, wenn man die Größe des den Kreis 1 durchfließenden Stromes durch zum Beispiel eine Änderung der Leistung der Stromquelle 2 oder der Größe des ohmschen Widerstands des Kreises 1 ändert. Dadurch kann man die Potenzen von herzustellenden Homöopathie -Präparaten regeln; man kann auch Präparate mit einer höheren Potenz gegenüber den ausgänglichen Präparaten, die als Informationsquelle dienen, herstellen. Indem man zum Beispiel eine experimentelle Stromgröße im Kreis 1 wählt, und als Quelle ein Homöopathie -Präparat mit einer Potenz von D10 benutzt, könnte man einen diesem entsprechenden Träger bekommen, der sich durch eine Potenz von D1000 und höher auszeichnet.

Die Ausgestaltung des Kontaktelements, das sich zum Zusammenwirken mit einer Informationsquelle eignet, in Form eines Trichters 14 gestattet es, die Intensität der Informationsabnahme, die sich an der Spitze des Trichters 14 konzentriert und über die Stromabnahmestelle 15 in den Kreis1 kommt, zu erhöhen. Das Kontaktelement in Form eines Trichters 14 ist ebenfalls bekannt, wenn als Informationsquelle das Akupunktursystem des Menschen dient, weil es gestattet, die ganze Zone des Akupunktursystems zu umfassen.

Die Variante der Einrichtung zum Energie-Informationsaustausch, die in Fig.7, 8 dargestellt ist, eignet sich zur Korrektur des Zustandes des Akupunktursystems des Menschen.

Diese Variante der Einrichtung arbeitet folgendermaßen:

Es ist bekannt, dass,wenn ein Organismus an einer Krankheit leidet, bestimmte Zonen des Akupunktursystems durch die Strahlung einer "pathologischen" Energie charakterisiert werden, die dieser Erkrankung eigen sind. Man bringt dann das die Form eines Trichters 14 aufweisende Kontaktelement zu dieser Zone in einen Abstand von 2 bis 3cm und schaltet das Gerät mittels des Schalters 7 ein. Die Strahlung von der Pathologiezone wird vom Trichter 14 aufgenommen und dem Kreis 1 übertragen. Die "Pathologiezone" konzentriert sich dabei um die Achse der Induktionsspule 4 und über den Stab 10 nach dem Leiter 16, kommt verstärkt in den Kreis 1 zurück und strahlt über den Trichter 14 auf dieselbe Zone des Akupunktursystems aus. Die Expositionszeit beträgt 1 bis 10-15 Minuten in Abhängigkeit von der Intensität des pathologischen Prozeßes.

Sich von der Reaktion des Kranken oder von den Ergebnissen der Elektropunkturdiagnostik leiten lassend wählt der Arzt die Parameter des Kreises 1, indem er zum Beispiel die Größe des Widerstands 17 ändert, bis positive Ergebnisse des Zustandes der Prüfakupunkturpunkte erreicht werden.

Ein elektrischer Kontakt des Stabes 10 mit dem Kreis 1 kann in verschiedenen Punkten des letzteren erzielt werden, was den Charakter der Einwirkung des vom Trichter 14 auszustrahlenden Energie-Informationsstromes (aufregend, bremsend) bestimmt.

Während der Verschiebung des Stabes 10 längs seiner Achse wird die Anzahl der Windungen, die eingesetzt werden, geändert. Dadurch wird ebenfalls die Regelung des Charakters der Einwirkung der Ausstrahlung auf das Akupunktursystem gewährleistet.

Indem man das Filter auf die Frequenz der Ausstrahlung der "pathologischen" Energie einstellt, kann man eine selektive Einwirkung der Energie-Informationsausstrahlung gerade auf das kranke Organ richten.

Die Verstärkung der Einwirkungseffektivität gestattet es, den Generator 18 einzusetzen . Dabei wählt man die Ausstrahlungsfrequenz des Generators 18 unter Berücksichtigung derselben Forderungen, die oben in bezug auf die Wahl der Parameter des Filters 19 dargelegt waren, aus.

Die Einstellung des Filters 19 und der Frequenz des Generators 18 wird praktisch nach der bekannten Methode der Akupunkturdiagnostik nach R.Voll vorgenommen.

Der Verstärker des Energie-Informationssignals, dessen elektrische Schaltung in Fig.9 dargestellt ist, arbeitet so, wie dies oben in bezug auf die Fig.1 oder 2 beschrieben wurde, mit einem Unterschied, daß jede zusätzliche Stufe eine zusätzliche Verstärkung des Energie-Informationssignals mit sich bringt, was zu einer Beschleunigung des Energie--Informationsaustausches beiträgt, die Zeit der Übertragung der Heileingenschaften der ausgänglichen Arzneimittel auf die Träger wesentlich verkürzt sowie die Qualität der Übertragung erhöht.

Das erfindungsgemäße Gerät kann zum Beispiel zur Übertragung teurer chemischer Reaktionsmittel auf billige Träger sowie zur Schaffung von Trägern, die einen breiteren Anwendungsbereich gegenüber Ausgangsreaktionsmitteln aufweisen, eingesetzt werden.

Die Einrichtung kann auch in der Medizin zur Erweisung einer Heilwirkung auf das Akupunktursystem sowie zur Herstellung von Arzneimitteln durch Übertragung der ausgänglichen Arzneimittel auf Träger eingesetzt werden.

Es ist vorteilhaft, das erfindungsgemäße Gerät zur Herstellung von Präparaten mit verschiedenen Potenzen anzuwenden.

## Patentansprüche

1. Vorrichtung zum Energieinformationsaustausch zwischen Objekten, von denen eines eine informative Substanz und das andere ein Informationsempfänger ist, enthaltend einen elektrischen Schaltkreis (1) mit einer Stromquelle (2), und ein erstes und ein zweites an den elektrischen Schaltkreis (1) angeschlossenes Kontaktelement (3, 4), wobei das erste zum Zusammenwirken mit der informativen Substanz und das zweite zum Zusammenwirken mit dem Informationsempfänger geeignet ist,
**dadurch gekennzeichnet,**
**dass** das zum Zusammenwirken mit dem Informationsempfänger (6) geeignete Kontaktelement (4) als eine in den Schaltkreis (1) eingeschaltete Induktionsspule in Form eines Torus ausgebildet ist.

2. Vorrichtung nach Anspruch 1, **dadurch gekennzeichnet, dass** die Induktionsspule (4a) mindestens eine zusätzliche Wicklung (13) aufweist, deren Windungen zur Grundwicklung (12) koaxial angeordnet sind.

3. Vorrichtung nach Anspruch 1, **dadurch gekennzeichnet, dass** die Induktionsspule (4a) mit einem Kern in Form eines Stabes (10) versehen ist und entlang der Drehachse der Induktionsspule (4a) angeordnet ist.

4. Vorrichtung nach Anspruch 3, **dadurch gekennzeichnet, dass** das Arbeitsende (11) des Stabes (10) spitz ausgebildet ist.

5. Vorrichtung nach Anspruch 3, **dadurch gekennzeichnet, dass** der Stab (10) mit dem Schaltkreis (1) elektrisch verbunden ist.

6. Vorrichtung nach Anspruch 5, **dadurch gekennzeichnet, dass** der Stab (10) entlang seiner Achse verschiebbar angeordnet ist.

7. Vorrichtung nach einem der Ansprüche 3 bis 6, **dadurch gekennzeichnet, dass** das Material des Stabes (10) unter Berücksichtigung seiner Verwandtschaft mit dem Material eines für die Behandlung der gegebenen Erkrankung angezeigten Homöopathie-Präparate ausgewählt ist.

## Claims

1. A device for energo-information exchange between objects, the first of which is a source of information, the second is a receiver of information, comprising an electric circuit (1) including power supply (2), the first and the second contact elements (3, 4) adapted for interaction, namely the first one with the source of information and the second one with the receiver of information, and connected to the circuit (1), **characterized in that** the contact element adapted for interaction with the receiver of information is formed as a toroidal inductance coil (4) included in the circuit (1).

2. A device according to claim 1, **characterized in that** the inductance coil (4a) comprises at least one additional winding (13) with winds placed coaxially to the conductor of winding (12).

3. A device according to claim 1, **characterized in that** the inductance coil (4a) comprises a core formed as a rod (10) placed on the rotation axes of the coil (4a).

4. A device according to claim 3, **characterized in that** the rod (10) comprises a pointed end (11).

5. A device according to claim 3, **characterized in that** the rod (10) is electrically connected in the circuit (1).

6. A device according to claim 5, **characterized in that** the rod (10) is placed movably along the axis thereof.

7. A device according to claims 3 and 5, **characterized in that** the rod (10) material is chosen in view of its relationship to the material of one of homeopathic preparations intended for treatment of a certain disease.

## Revendications

1. Dispositif pour échange d'énergoinformation entre les objets, l'un desquels est une source d'information et l'autre est un récepteur d'information, comprenant un contour électrique (1) contenant la source d'alimentation (2), le premier et le deuxième éléments de contact (3, 4), adaptés à coopérer l'un avec la source d'information et l'autre avec le récepteur d'information, et branchés sur le contour électrique (I), **caractérisé en ce que** l'élément de contact, adapté à coopérer avec le récepteur d'information, représente une bobine d'induction (4) de forme toroïdale insérée dans le contour électrique (I).

2. Dispositif selon la revendication 1, **caractérisé en ce que** la bobine d'induction (4a) est pourvue d'au moins un enroulement supplémentaire (13), les spires duquel sont placées coaxialement par rapport au conducteur de l'enroulement (12).

3. Dispositif selon la revendication 1, **caractérisé en ce que** la bobine d'induction (4a) est pourvue d'un noyau, ayant la forme d'un tige (10) disposé suivant l'axe de rotation de la bobine (4a).

4. Dispositif selon la revendication 3, **caractérisé en ce. qu'**un bout (11) du tige (10) est exécuté pointu.

5. Dispositif selon la revendication 3, **caractérisé en ce que** le tige (10) est électriquement relié au contour (1).

6. Dispositif selon la revendication 5, **caractérisé en ce que** le tige (10) est monté déplaçable le long de son axe.

7. Dispositif selon l'une des revendications 3 et 5, **caractérisé en ce que** le matériel duquel le tige (10) est exécuté est choisi en tenant compte de son affinité avec le matériel d'une des préparations homéopathiques indiquées pour le traitement de cette maladie.
